# EUROPÄISCHE PATENTSCHRIFT

(11) **EP 1 123 329 B1**
(45) Veröffentlichungstag und Bekanntmachung des Hinweises auf die Patenterteilung: **15.09.2004**
(21) Anmeldenummer: 99947451.3
(22) Anmeldetag: 01.10.1999
(51) Int. Cl.: C08F 251/00, C08F 261/04, C08F 283/06, C08F 283/02, C08F 220/06, A61L 15/00, A61F 13/15

(54) **HYDROPHILE, HOCHQUELLFÄHIGE HYDROGELE SOWIE VERFAHREN ZU IHRER HERSTELLUNG UND VERWENDUNG**
HYDROPHILIC HYDROGELS WITH A HIGH SWELLING CAPACITY AND METHOD FOR PRODUCING AND USING THEM
HYDROGELS HYDROPHILES A FORTE CAPACITE DE GONFLEMENT ET PROCEDE PERMETTANT DE LES PREPARER ET DE LES UTILISER

(30) Priorität: 08.10.1998 DE 19846412
(43) Veröffentlichungstag der Anmeldung: 16.08.2001
(73) Patentinhaber: BASF AKTIENGESELLSCHAFT, 67056 Ludwigshafen (DE)
(72) Erfinder: DENTLER, Joachim, D-63486 Bruchköbel (DE); FUNK, Rüdiger, D-65527 Niedernhausen (DE); HERFERT, Norbert, D-63674 Altenstadt (DE); WANIOR, Mariola, D-63526 Erlensee (DE); ENGELHARDT, Friedrich, Chesapeake, VA 23320 (US)
(86) Internationale Anmeldenummer: PCT/EP1999/007308
(87) Internationale Veröffentlichungsnummer: WO 2000/022018

(56) Entgegenhaltungen:
- WO-A-97/06190
- US-A- 4 985 514
- US-A- 5 747 570

## Beschreibung

Die vorliegende Erfindung betrifft hydrophile, hochquellfähige Hydrogele mit hohem Aufnahmevermögen für Wasser und wäßrige Flüssigkeiten, ihr Herstellungsverfahren und die Verwendung dieser Hydrogele.

Hydrophile Hydrogele, die durch Polymerisation ungesättigter Säuren, wie beispielsweise Acrylsäure, Methacrylsäure, Acrylamidopropansulfonsäure usw., in Gegenwart geringer Mengen mehrfach olefinisch ungesättigter Verbindungen erhalten werden können, sind bereits als superabsorbierende Polymere bekannt. Weiterhin sind auch hydrophile Hydrogele bekannt, die durch Pfropfcopolymerisation olefinisch ungesättigter Säuren auf unterschiedliche Matrices, wie beispielsweise Polysaccharide, Polyalkylenoxide sowie deren Derivate, zugänglich sind.

Die genannten Hydrogele zeichnen sich durch ein hohes Aufnahmevermögen für Wasser und wäßrige Lösungen aus und finden daher bevorzugt Anwendung als Absorptionsmittel in Hygieneartikeln.

Die Herstellung solcher wasserquellbarer hydrophiler Polymerer erfolgt in der Regel durch radikalische Polymerisation in wäßriger Lösung, die die Monomeren, sowie gegebenenfalls Pfropfgrundlage und Vernetzer enthält.

Für die Verwendung der wasserquellbaren hydrophilen Polymere in Hygiene und Sanitärbereich werden Polymerisate erzeugt, deren Neutralisationsgrad zwischen 50 und 85 Mol-% bezogen auf die polymerisierten, Säuregruppen enthaltenden Monomereinheiten, beträgt, so daß bei der Verwendung hautneutral wirkende Hydrogele gebildet werden.

Die Einstellung des Neutralisationsgrades wird in der Regel vor der Polymerisation vorgenommen, da so die technisch schwierig durchzuführende Neutralisation eines sauren, hochviskosen Hydrogels umgangen wird. Nun zeigt aber die Polymerisation von z.B. Acrylsäure im neutralen pH-Bereich eine niedrigere Polymerisationsgeschwindigkeit und führt zu niedrigeren Molekulargewichten als die Polymerisation im sauren Bereich. Dies wird durch die elektrostatische Abstoßung zwischen der zuletzt eingebauten Monomereinheit und der neu einzubauenden Monomereinheit erklärt, die im Falle der Polymerisation im sauren pH-Bereich nicht oder nur gering auftritt, da die Monomereinheiten in der ungeladenen, sauren Form vorliegen.

Mit dem Trend zu immer dünner werdenden Windelkonstruktionen werden an die wasserquellbaren hydrophilen Polymeren zunehmend steigende Anforderungen hinsichtlich Absorptionsvermögen, Flüssigkeitsaufnahme und Flüssigkeitstransport innerhalb des Hygieneartikels, insbesondere auch unter Druckbelastung, gestellt.

In der EP-A-0 640 330, WO 95/22358, WO 95/26209 und WO 97/12575 wird ein Test zur Messung der Gelpermeabilität von gequollenen Hydrogelpartikeln beschrieben (Saline Flow Conductivity, SFC). Hierbei wird der Durchfluß einer Kochsalz-Lösung durch eine vorgequollene Hydrogel-Partikelschicht unter einem Druck von 0.3 psi bestimmt. Da bei dieser Testmethode die Kochsalz-Lösung in Richtung der Schwerkraft durch die gequollene Gelschicht hindurchfließt, ist diese Methode wenig geeignet, auftretende Kapillarkräfte zu charakterisieren. In der Praxis ist für die Qualität von Hygieneartikeln jedoch bedeutsam, daß Flüssigkeit unter Druckbelastung auch entgegen der Schwerkraft transportiert werden kann, d.h. hier spielen Kapillarkräfte eine wichtige Rolle.

Es bestand daher die Aufgabe, hydrophile, hochquellfähige Hydrogele mit verbesserten Absorptionseigenschaften, insbesondere mit verbesserter Kapillarität unter Druckbelastung bereitzustellen.

Diese Aufgabe wird gelöst durch ein hydrophiles, hochquellfähiges Hydrogel auf Basis (co)polymerisierter Monomerer oder auf Basis von Propf(co)polymerer, erhältlich durch
a) radikalische (Co)polymerisation von einem oder mehreren hydrophilen Monomeren der Formel (I) oder Pfropf(co)polymerisation von einem oder mehreren hydrophilen Monomeren der Formel (I) auf eine Pfropfgrundlage, wobei der durchschnittliche Neutralisationsgrad der säuregruppenhaltigen Monomeren 0 bis 40 Mol-% beträgt
b) Zerkleinerung des sauren Hydrogels
c) Neutralisation des sauren Hydrogels auf einen End-Neutralisationsgrad von 50 - 85 mol-% durch Zugabe eines Neutralisationsmittels
d) Trocknung, Mahlung und Siebung der Hydrogelteilchen,
wobei das Hydrogel
- eine Zentrifugenretention für eine 0,9 %ige wäßrige NaCl-Lösung von mindestens 29 g/g und
- eine Quellhöhe bei der Vertikalabsorption (1 g) von mindestens 3,5 cm
- einen Gehalt an Extrahierbaren (16 h-Wert) kleiner 5 % aufweist
oder
- eine Zentrifugenretention für eine 0,9 %ige wäßrige NaCl-Lösung von mindestens 23 g/g und
- eine Quellhöhe bei der Vertikalabsorption (3 g) von mindestens 5 cm
- einen Gehalt an Extrahierbaren (16 h-Wert) kleiner 4 % aufweist

Die erfindungsgemäßen hydrophilen, hochquellfähigen Hydrogele sowie das Verfahren zu ihrer Herstellung werden im folgenden erläutert.

Zur Herstellung der erfindungsgemäßen, wasserquellbaren hydrophilen Polymeren geeignete hydrophile Monomere sind beispielsweise polymerisationsfähige Säuren, wie Acrylsäure, Methacrylsäure, Vinylsulfonsäure, Vinylphosphonsäure, Styrolsulfonsäure, Maleinsäure einschließlich deren Anhydrid, Fumarsäure, Itaconsäure, 2-Acrylamido-2-methylpropansulfonsäure, 2-Acrylamido-2-methylpropanphosphonsäure sowie deren Amide, Hydroxyalkylester, aminogruppen- oder ammoniumgruppenhaltige Ester und Amide. Des weiteren geeignet sind wasserlösliche N-Vinylamide oder auch Diallyldimethylammoniumchlorid.

Die hydrophilen Monomere sind Verbindungen der allgemeinen Formel (I) 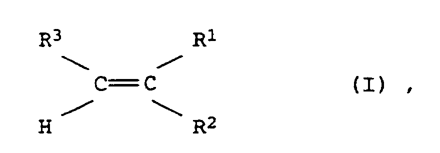 worin
- R¹: Wasserstoff, Methyl oder Ethyl,
- R²: eine Gruppe -COOR⁴, Hydroxysulfonyl, Phosphonyl, die mit (C₁ - C₄)-Alkanol veresterte Phosphonylgruppe oder eine Gruppe der allgemeinen Formel (II) 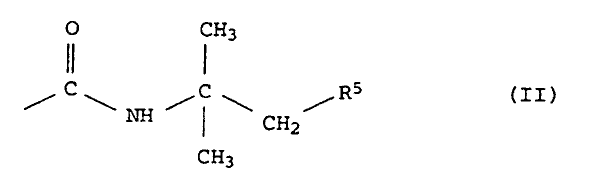
- R³: Wasserstoff, Methyl, Ethyl oder die Carboxylgruppe,
- R⁴: Wasserstoff, Amino-(C₁-C₄)-alkyl oder Hydroxy-(C₁-C₄)-alkyl und
- R⁵: Hydroxysulfonyl, Phosphonyl oder Carboxyl bedeuten.

Beispiele für (C₁-C₄)-Alkanole sind Methanol, Ethanol, n-Propanol oder n-Butanol.

Besonders bevorzugte hydrophile Monomere sind Acrylsäure und Methacrylsäure.

Sofern es sich bei den Monomeren um Säuren handelt, sind bis zu einem Anteil von 40 Gew.-% auch deren Alkalimetall- oder Ammoniumsalze als Comonomere geeignet.

Geeignete Pfropfgrundlagen können natürlichen oder synthetischenUrsprungs sein. Beispiele sind Stärke, Cellulose oder Cellulosederivate sowie andere Polysaccharide und Oligosaccharide, Polyvinylalkohol, Polyalkylenoxide, insbesondere Polyethylenoxide und Polypropylenoxide, sowie hydrophile Polyester. Geeignete Polyalkylenoxide haben beispielsweise die Formel (III) 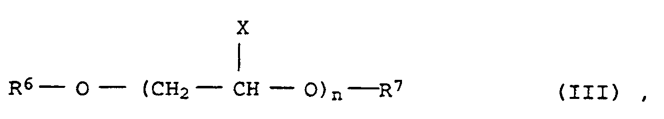 worin
- R⁶ und R⁷: unabhängig voneinander Wasserstoff, Alkyl, Alkenyl oder Aryl,
- X: Wasserstoff oder Methyl und
- n: eine ganze Zahl von 1 bis 10000 bedeuten.

Rest R⁶ und R⁷ sind beispielsweise lineares oder verzweigtes (C₁-C₁₀)-Alkyl wie Methyl, Ethyl, Propyl, i-Propyl, n-Butyl, (C₂-C₆)-Alkenyl oder Aryl wie gegebenenfalls mit (C₁-C₄)-Alkyl substituiertes Phenyl.

R⁶ und R⁷ bedeuten bevorzugt Wasserstoff, (C₁-C₄)-Alkyl, (C₂-C₆)-Alkenyl oder Phenyl.

Die hydrophilen, hochquellfähigen Hydrogele sind bevorzugt vernetzt, d.h. sie enthalten Verbindungen mit mindestens zwei Doppelbindungen, die in das Polymernetzwerk einpolymerisiert sind.

Geeignete Vernetzer sind insbesondere Methylenbisacryl- bzw. -methacrylamid, Ester ungesättigter Mono- oder Polycarbonsäuren von Polyolen, wie Diacrylat oder Triacrylat, z.B. Butandiol- oder Ethylenglykoldiacrylat bzw. -methacrylat sowie Trimethylolpropantriacrylat, Allylverbindungen wie Allyl(meth)acrylat, Triallylcyanurat, Maleinsäurediallylester Polyallylester, Tetraallyloxyethan, Triallylamin, Tetraallylethylendiamin, Pentaerythritoltriallylester oder Allylester der Phosphorsäure sowie Vinylverbindungen wie Vinylacrylat, Divinyladipinat, Divinylbenzol und Vinylphosphonsäurederivate, wie sie beispielsweise in der EP-A-0 343 427 beschrieben sind.

Zur Auslösung der Polymerisation können energiereiche elektromagnetische Strahlen oder die üblichen chemischen Polymerisationsinitiatoren herangezogen werden, z.B. organische Peroxide, wie Benzoylperoxid, tert.-Butylhydroperoxid, Methylethylketonperoxid, Cumolhydroperoxid, Azoverbindungen wie Azodiisobutyronitril sowie anorganische Peroxiverbindungen wie Ammoniumpersulfat, Kaliumpersulfat oder Wasserstoffperoxid, gegebenenfalls in Kombination mit Reduktionsmitteln wie Natriumhydrogensulfit, und Eisen(II)-sulfat oder Redoxsystemen, welche als reduzierende Komponente eine aliphatische und aromatische Sulfinsäure, wie Benzolsulfinsäure und Toluolsulfinsäure oder Derivate dieser Säure enthalten, z.B. Mannichaddukte aus Sulfinsäure, Aldehyden und Aminoverbindungen.

Bevorzugt ist die Polymerisation in wäßriger Lösung nach der sogenannten Gel-Polymerisation unter Ausnutzung des Trommsdorff-Norrish-Effektes. Besonders bevorzugt ist, die Polymerisation in Ruhe ohne mechanische Durchmischung durchzuführen, so daß keine mechanischen Scherkräfte auf das sich bildende Hydrogel einwirken, die zu einer Erhöhung des Gehaltes an Extrahierbaren führen würden. Die Polymerisation kann hierbei sowohl diskontinuierlich, z.B. in einem zylindrischen Reaktor, oder kontinuierlich, z.B. durch Polymerisation auf einem Bandreaktor, durchgeführt werden.

Die Grobzerkleinerung der erhaltenen Hydrogele erfolgt mittels üblicher Reiß- und/oder Schneidwerkzeuge, z.B. durch die Wirkung einer Austragspumpe im Falle der Polymerisation in einem zylindrischen Reaktor oder durch eine Schneidwalze oder Schneidwalzenkombination im Falle der Bandpolymerisation.

Auf diese Weise erhält man saure Hydrogel-Teilchen, die auf den gewünschten Endneutralisationsgrad von 50 - 85 Mol-% bezogen auf Säuregruppen tragende Monomereinheiten durch Neutralisation dieser Säuregruppen tragenden Monomereinheiten gebracht werden müssen. Diese Neutralisation ist ein technisch schwierig durchzuführender Prozeß, an den besondere Anforderungen gestellt werden. Zum einen darf das Gel bei dem Inkontaktbringen mit dem Neutralisationsmittel nicht zu sehr geschert werden, um einen Anstieg der Extrahierbaren zu vermeiden, der sich negativ auf die Produkteigenschaften des Endproduktes auswirkt und somit unerwünscht ist, zum anderen muß die Neutralisation vollständig homogen erfolgen.

Die nachträgliche Neutralisation von sauren Hydrogelen ist prinzipiell bekannt.

Die DE-A-26 12 846 offenbart ein Verfahren zur Herstellung eines wasserabsorbierenden Harzes, wobei wenigstens eine Stärke und/oder Cellulose mit wenigstens einem wasserlöslichen Monomeren mit einer polymerisierbaren Doppelbindung und einem Vernetzer polymerisiert wird. Die erhaltenen Polymeren werden mit Alkalien neutralisiert, wobei das Verfahren zur Neutralisation nicht näher spezifiziert ist.

Nach der EP-A-0 205 674 werden saure Polymerisate bei Temperaturen im Bereich von 0 bis 100°C, vorzugsweise 5 bis 40°C hergestellt, deren pH durch nachträgliche Teilneutralisation des Hydrogels eingestellt wird. Die Neutralisation wird hierbei durch Zugabe des Gels in eine sehr stark verdünnte Natronlauge erzielt. Dieses Verfahren ist nachteilig, da bei der Trocknung durch die starke Verdünnung der Natronlauge große Wassermengen verdampft werden müssen.

In der EP-A-0 303 440 wird die Herstellung eines hydratisierten, vernetzten Gelpolymers beschrieben, bei dem die Säuregruppen enthaltenden Monomeren zu 10 bis 50 Mol-% neutralisiert sind, und welches durch Zugabe eines Neutralisationsmittels unter Nutzung der Scherkräfte der Rührblätter eines Reaktionsgefäßes mit einer Vielzahl von rotierenden Wellen auf den gewünschten Endneutralisationsgrad eingestellt wird. Nach diesem Verfahren wird zwar eine homogene Neutralisation erzielt, da ständig neue Oberfläche der Gelteilchen erzeugt wird, die Scherbelastung des Gels ist jedoch zu hoch und führt zum unerwünschten Anstieg der extrahierbaren Anteile.

Die EP-A-0 238 050 beansprucht ein Verfahren zur diskontinuierlichen Herstellung von vernetzten, feinteiligen, wasserabsorbierenden Polymerisaten, wobei die Polymerisation im Kneter durchgeführt wird und der Neutralisationsgrad der (Meth)acrylsäure zwischen 0 und 100 Mol-% liegt. Die Neutralisation des Polymerisationsansatzes zum gewünschten End-pH erfolgt ebenfalls in dem auch als Polymerisationsreaktor benutzen Kneter, und zwar entweder noch während der Polymerisation oder im Anschluß an die Polymerisationsreaktion. Auch hier wird zwar eine homogene Neutralisation erzielt, die aufgewendeten Scherkräfte sind jedoch zu hoch, so daß es zu einem unerwünschten Anstieg der Extrahierbaren kommt.

Nach der US-5 453 323 und EP-A-0 530 438 werden ohne Neutralisation der Monomeren aus Acrylsäure zusammen mit wasserlöslichen Hydroxylgruppen enthaltenden Polymeren unter adiabatischen Bedingungen Polymerisatgele hergestellt, die anschließend in einem nicht weiter spezifizierten Fleischwolf zerkleinert werden. Zu diesem zerkleinerten Gel wird das Neutralisationsmittel gegeben und das Gemisch wird erneut gewolft. Anschließend erfolgt die Zugabe eines Nachvernetzungsmittels und eine erneute dreimalige Wolfung des Gels, um alle Reaktanden homogen in das Gel einzuarbeiten. Durch diese mehrmalige Wolfung des Gels findet eine unerwünschte Scherbelastung des Gels statt, die zu erhöhten Anteilen von Extrahierbaren führt.

Die EP-A-0 629 411 beschreibt die die Polymerisation von Acrylsäure mit Vernetzer, wobei das erhaltene Gel anschließend mit einem Alkalisalz teilneutralisiert und durch Zugabe eines Vernetzungsmittels weiter vernetzt wird. Das Verfahren zur Neutralisation ist in der Schrift nicht näher spezifiziert, in einem Beispiel wird das Verkneten des Gels mit dem Neutralisationsmittel in einem Extruder erwähnt.

In der DE-A-195 29 348 ist zur Herstellung von superabsorbierenden Polymeren die adiabatische Polymerisation einer teilvorneutralisierten Monomerlösung beschrieben, wobei der Vorneutralisationsgrad der säuregruppentragenden Monomeren 5 bis 30 Mol-% beträgt. Die Neutralisation des sauren Gels erfolgt nach dessen Zerkleinerung in einfachen Mischaggregaten wie in einer rotierenden Trommel oder in einem Draismischer, wobei die wäßrige Lösung der Basen beispielsweise über Düsen oder Sprühlanzen eingetragen wird. Auf diese Weise wird zwar eine mechanische Schädigung des Polymergels vermieden, es kann aber keine homogene Neutralisation erzielt werden, da bei der Vermischung mit dem Neutralisationsmittel das Gel nicht aufgeschlossen wird. Die pH-Inhomogenitäten des Gels bewirken wiederum ein verschlechtertes Trocknungsverhalten, welches aus wirtschaftlichen Gründen unerwünscht ist.

Das saure Hydrogel wird bevorzugt neutralisiert, indem es zusammen mit dem Neutralisationsmittel in einem Wolf mittels eines Systems aus Schnecke, rotierendem Messer, Stauzone und Lochscheibe mit einer Leistung von 1000 bis 6000 Wh/m³, bevorzugt von 2500 bis 5000 Wh/m³, aufgeschlossen und vermischt wird, wobei das Hydrogel durch eine Zone mit einer Energiedissipationsdichte von 400 bis 800 W/l Mischvolumen geführt wird. Das Verfahren arbeitet mit Verweilzeiten von 5 bis 30 Sekunden- Die Frequenz des rotierenden Messers beträgt 1 - 5 s⁻¹, bevorzugt 3 - 4 s⁻¹. Zur Reduzierung der Scherkräfte beim Mischvorgang im Stauraum vor der Lochscheibe des Apparates sind die Bohrungen der Lochscheibe konisch ausgeführt. Die freie Lochfläche der Lochscheibe beträgt 20 bis 40 %, vorzugsweise 25 bis 35 %, der Lochanfangsdurchmesser 4 bis 16 mm, bevorzugt 8 bis 10 mm bei einer konischen Erweiterung unter einem Winkel von 8° bis 20°, vorzugsweise 10° bis 15°. Ein Wolf ähnelt apparativ einem Extruder, übt aber geringere Scherkräfte aus.

Die beschriebene Konstruktion erlaubt die Kombination aus hoher Mischgüte und schonender mechanischer Beanspruchung des Gemisches aus Hydrogel und Neutralisationsmittel. Eine einstufige Behandlung erweist sich hierbei als absolut ausreichend für eine homogene Neutralisation, so daß ein mehrmaliges Wolfen des Gels vermieden wird, welches wiederum zu einem Anstieg der Scherbelastung des Gels führen würde, welcher unerwünscht ist.

Die Wahl des Neutralisationsmittels ist unkritisch, geeignete Neutralisationsmittel sind Alkalihydroxide, Ammoniak, aliphatische primäre und sekundäre Amine, Alkalicarbonate und Alkalihydrogencarbonate. Besonders bevorzugt sind Natriumhydroxid und Natriumcarbonat. Die Zugabe des Neutralisationsmittels kann sowohl in flüssiger Form, z.B. Natronlauge, in fester Form, z.B. Natriumcarbonat-Pulver oder gasförmig, z.B. Ammoniak, erfolgen.

Bei der erfindungsgemäßen Durchführung der Neutralisation ist es aufgrund der speziellen Konstruktion des Wolfes auch möglich, zusätzlich andere Reaktanden bzw. Stoffe mit dem zu neutralisierenden Polymergel zu vermischen. Durch dieses Verfahren wird ein mehrstufiges Wolfen vermieden, welches auf unerwünschte Weise zu einer mechanischen Scherbelastung des Gels führen würde.

So können dem Gel Reaktanden zugegeben werden, die mit freier Acrylsäure reagieren können, wie zum Beispiel Aminosäuren wie Cystein oder Lysin, Hydroxylamin und/oder seine Salze wie Hydrochlorid oder Sulfat, Hydrazin und/oder dessen Salze, Ozon oder schwefelhaltige Verbindungen mit reduzierender Wirkung, wie Alkalisulfite, -hydrogen-sulfite oder -disulfite, Natriumthiosulfat oder Mercaptoverbindungen.

Es können dem Gel auch Stoffe zugegeben werden, die mit den Carboxylgruppen des Hydrogels unter Vernetzung reagieren können. Beispiele für solche Stoffe sind mehrwertige Alkohole, mehrwertige Amine, Polyamidoamine und deren Umsetzungsprodukte mit Epichlorhydrin, Di- und Polyepoxide, Bis- und Polyaziridine, Bisund Polyoxazoline, Di- und Polyisocyanate, Ethylencarbonat oder Oxazolidon.

Des weiteren ist es möglich, das Gel in dieser Stufe mit Feinanteilen superabsorbierender Polymere zu mischen, die beispielsweise bei der Herstellung von wasserquellbaren hydrophilen Hydrogelen bei der Mahlung und anschließenden Absiebung der getrockneten Hydrogele anfallen.

Die Neutralisation des sauren Hydrogels kann auch nach einem zweistufigen Verfahren erfolgen. Hierbei wird in der ersten Stufe in einem Wolf, der die zuvor beschriebenen Bedingungen erfüllt, ein Neutralisationsgrad von mindestens 50 Gew.-%, bevorzugt von mindestens 55 Gew.-% und besonders bevorzugt von mindestens 60 Gew.-% eingestellt. In der zweiten Stufe wird durch Aufsprühen eines Neutralisationsmittels bzw. dessen wäßriger Lösung auf die Hydrogelpartikel, d.h. ohne mechanische Scherbelastung der Gelpartikel, der Neutralisationsgrad auf den gewünschten End-Neutralisationsgrad erhöht. Das Neutralisationsmittel im zweiten Schritt kann gleich oder verschieden dem Neutralisationsmittel im ersten Schritt sein. Bevorzugt wird für den zweiten Neutralisationsschritt wäßrige Natronlauge eingesetzt.

Zur Trocknung der Hydrogelteilchen sind verschiedene Verfahren bekannt. So kann die Trocknung beispielsweise nach dem Dünnfilm-Trockenverfahren, z.B. mit Hilfe eines Zwei-Achsen-Walzentrockners, nach dem Plattentrockenverfahren, gemäß dem die Hydrogelpolymerteilchen auf Platten in mehreren Schichten in einer Trockenkammer geladen werden, in der Heißluft zirkuliert, nach dem Drehtrommel-Verfahren mit Hilfe von Walzentrocknern oder nach dem Förderband-Verfahren, im folgenden auch als Bandtrocknung bezeichnet, erfolgen. Bevorzugt ist die Trocknung mit Hilfe von Walzentrocknern und die Bandtrocknung, bei der mit Löcher versehene Horden eines Kreisförderes in einem Tunnel mit Trocknungsgut beladen und das Trocknungsgut während der Förderung durch Durchblasen von Heißluft durch die Hordenlöcher getrocknet wird.

Das getrocknete Hydrogel wird gegebenenfalls vorzerkleinert und dann gemahlen, wobei die Mahlung bevorzugt mit Hilfe eines Walzenstuhls erfolgt, um den Anfall an Feinteilen möglichst klein zu halten. Bei der anschließenden Siebung wird die Korngrößenverteilung eingestellt, die in der Regel zwischen 100 und 1000 µm, bevorzugt zwischen 120 und 850 µm, liegt. Zu grobe Partikel können erneut der Mahlung unterworfen werden, zu feinteilige Partikel können dem Herstellungsprozeß zurückgeführt werden, beispielsweise durch Vermischung mit dem zu neutralisierenden Gel bei dem Neutralisationsschritt im Wolf, oder für getrennte Verwendungszwecke eingesetzt werden.

In einer bevorzugten Ausführung der Erfindung werden die Absorptionseigenschaften der so erhaltenen hydrophilen, hochquellfähigen Hydrogele durch eine anschließende Oberflächennachvernetzung noch weiter verbessert. Hierzu werden Verbindungen, die mit den Carboxylgruppen des Hydrogels unter Vernetzung reagieren können, vorzugsweise in Form einer wasserhaltigen Lösung auf die Oberfläche der Hydrogel-Partikel aufgebracht. Geeignete Nachvernetzungsmittel sind beispielsweise Di- oder Polyglycidylverbindungen wie Phosphonsäurediglycidylether oder Ethylenglykoldiglycidylether, Alkoxysilylverbindungen, Polyaziridine, Polyamine oder Polyamidoamine sowie deren Umsetzungsprodukte mit Epichlorhydrin, Polyole wie Ethylenglykol, 1,2-Propandiol, 1,4-Butandiol, Glycerin, Di- und Polyglycerin, Pentaerythrit, Sorbit, die Oxethylate dieser Polyole sowie deren Ester mit Carbonsäuren oder der Kohlensäure, Ethylencarbonat, Propylencarbonat, Oxazolidon, Bisoxazolin, Polyoxazoline, Di- und Polyisocyanate. Bei Bedarf können saure Katalysatoren wie beispielsweise p-Toluolsulfonsäure, Phosphorsäure, Borsäure oder Ammonium-dihydrogenphosphat zugesetzt werden.

Geeignete Mischaggregate zum Aufsprühen der Vernetzer-Lösung auf die Hydrogel-Partikel sind beispielsweise Patterson-Kelly-Mischer, DRAIS-Turbulenzmischer, Lödige-Mischer, Schneckenmischer, Tellermischer, Wirbelschichtmischer, Schugi-Mischer. Nach Aufsprühen der Vernetzer-Lösung kann ein Temperaturbehandlungsschritt nachfolgen, bevorzugt in einem nachgeschalteten Trockner, bei einer Temperatur zwischen 80 und 230°C, bevorzugt 80 - 190°C, und besonders bevorzugt zwischen 100 und 160°C, über einen Zeitraum von 5 Minuten bis 6 Stunden, bevorzugt 10 Minuten bis 2 Stunden und besonders bevorzugt 10 Minuten bis 1 Stunde, wobei sowohl Spaltprodukte als auch Lösungsmittelanteile entfernt werden können.

In einer besonders bevorzugten Ausführung der Erfindung wird zusätzlich die Hydrophilie der Hydrogel-Partikeloberfläche durch Ausbildung von Metallkomplexen modifiziert. Die Bildung der Metallkomplexe auf der äußeren Schale der Hydrogel-Partikel erfolgt durch Aufsprühen von Lösungen zwei- oder mehrwertiger Metallsalz-Lösungen, wobei die Metall-Kationen mit den Carboxylgruppen des Hydrogels unter Ausbildung von Komplexen reagieren können. Beispiele für zwei- oder mehrwertige Metall-Kationen sind Mg²⁺, Ca²⁺, Al³⁺, Sc³⁺, Ti⁴⁺, Mn²⁺, Fe^{2+/3+}, Co²⁺, Ni²⁺, Cu^{+/2+}, Zn²⁺, Y³⁺, Zr⁴⁺, Ag⁺, La³⁺, Ce⁴⁺, Hf⁴⁺, und Au^{+/3+}, bevorzugte Metall-Kationen sind Mg²⁺, Ca²⁺, Al³⁺, Ti⁴⁺, Zr⁴⁺ und La³⁺, und besonders bevorzugte Metall-Kationen sind Al³⁺, Ti⁴⁺und Zr⁴⁺. Die Metall-Kationen können sowohl allein als auch im Gemisch untereinander eingesetzt werden. Von den genannten Metall-Kationen sind alle Metallsalze geeignet, die eine ausreichende Löslichkeit in dem zu verwendenden Lösungsmittel besitzen. Besonders geeignet sind Metallsalze mit schwach komplexierenden Anionen wie zum Beispiel Chlorid, Nitrat und Sulfat. Als Lösungsmittel für die Metallsalze können Wasser, Alkohole, DMF, DMSO sowie Mischungen dieser Komponenten eingesetzt werden. Besonders bevorzugt sind Wasser und Wasser/Alkohol-Mischungen wie zum Beispiel Wasser/Methanol oder Wasser/1,2-Propandiol.

Das Aufsprühen der Metallsalz-Lösung auf die Hydrogel-Partikel kann sowohl vor als auch nach der Oberflächennachvernetzung der Hydrogel-Partikel erfolgen. In einem besonders bevorzugten Verfahren erfolgt das Aufsprühen der Metallsalz-Lösung im gleichen Schritt mit dem Aufsprühen der Vernetzer-Lösung, wobei beide Lösungen getrennt nacheinander oder gleichzeitig über zwei Düsen aufgesprüht werden, oder Vernetzer- und Metallsalz-Lösung vereint über eine Düse aufgesprüht werden können.

Gegebenenfalls kann noch eine weitere Modifizierung der Hydrogel-Partikel durch Zumischung feinteiliger anorganischer Feststoffe, wie zum Beispiel Silica, Aluminiumoxid, Titandioxid und Eisen(II)-oxid erfolgen, wodurch die Effekte der Oberflächennachbehandlung noch weiter verstärkt werden. Besonders bevorzugt ist die Zumischung von hydrophilem Silica oder von Aluminiumoxid mit einer mittleren Größe der Primärteilchen von 4 bis 50 nm und einer spezifischen Oberfläche von 50 - 450 m²/g. Die Zumischung feinteiliger anorganischer Feststoffe erfolgt bevorzugt nach der Oberflächenmodifizierung durch Vernetzung/Komplexbildung, kann aber auch vor oder während diesen Oberflächenmodifizierungen durchgeführt werden.

Die erfindungsgemäßen Hydrogele zeichnen sich durch hervorragendes Absorptionsvermögen, hohe Kapillarität und niedrige Gehalte an Extrahierbaren aus und sind deshalb in hervorragender Weise als Absorptionsmittel für Wasser und wäßrige Flüssigkeiten, insbesondere von Körperflüssigkeiten, wie z.B. Urin oder Blut geeignet, beispielsweise in Hygieneartikeln wie z.B. Baby- und Erwachsenenwindeln, Damenbinden, Tampons und dergleichen. Sie können aber auch als Bodenverbesserungsmittel in Landwirtschaft und Gartenbau, als Feuchtigkeitsbindemittel bei der Kabelummantelung sowie zum Eindicken wäßriger Abfälle verwendet werden.

### Beschreibung der in den Beispielen verwendeten Testmethoden:

### CRC (Centrifuge Retention Capacity):

Zur Bestimmung der CRC werden 0,2 g Hydrogel (Kornfraktion 106 - 850 µm) in einen 60 x 85 mm großen Teebeutel eingewogen, der anschließend verschweißt wird. Der Teebeutel wird dann in einen Überschuß von 0,9 Gew.-%iger Kochsalz-Lösung gegeben (mindestens 0,83 l Kochsalz-Lösung/1 g Hydrogel). Nach 30 Minuten Quellzeit wird der Teebeutel aus der Kochsalz-Lösung genommen und bei 250 g drei Minuten lang zentrifugiert. Durch Wägung des zentrifugierten Teebeutels wird die von dem Hydrogel festgehaltene Flüssigkeitsmenge ermittelt.

### Extrahierbare Anteile (16 h):

Zur Bestimmung der extrahierbaren Anteile werden 1 g Hydrogel (Kornfraktion 106 - 850 µm) in 200 ml 0,9 Gew.-%ige Kochsalzlösung eingerührt. Das Becherglas wird abgedichtet und die Mischung 16 h lang gerührt. Danach wird durch einen 0,22 µm-Filter abfiltriert und der Gehalt an Extrahierbaren durch eine Säure-Base-Titration der Carboxylgruppen bestimmt (Titration mit 0,1 normaler NaOH bis pH 10, anschließend Titration mit 0,1 normaler HCl bis pH 2,7).

### Vertikal-Absorption:

Die Kapillarität wird mit Hilfe der Vertikal-Absorption bestimmt. Die Testapparatur besteht aus Meßzellen und einem Flüssigkeitsbehälter. Die Meßzellen stellen ein zylindrisches Plexiglas-Rohr mit einem Innendurchmesser von 2,6 cm und einer Länge von 15 cm dar. Das obere Ende des Rohres ist offen, das untere Ende des Rohres besitzt einen 36 µm-Siebboden. In Höhe von 3 cm (vom unteren Ende des Rohres) besitzt das Rohr einen Tragring. Der Flüssigkeitsbehälter ist ein Plexiglaskasten von 30,0 cm Länge, 20,5 cm Breite und 3,8 cm Höhe. Im Abstand von 1,5 cm von einer Querseite ist eine 2 cm hohe Überlaufwand eingebaut. Auf der gegenüberliegenden Seite befindet sich eine Verbindung zum Flüssigkeitsbehälter, so daß ein konstanter Flüssigkeitsspiegel gewährleistet ist. Der Plexiglaskasten besitzt einen abnehmbaren Dekkel, der mit 6 kreisrunden Löchern mit einem Durchmesser von jeweils 3,2 cm versehen ist. Zur Durchführung der Messung wird 1 g Hydrogel (Vertikalabsorption 1 g) bzw. 3 g Hydrogel (Vertikalabsorption 3 g) in eine Meßzelle eingewogen, wobei die Hydrogel-Partikel gleichmäßig auf dem Siebboden verteilt werden. Anschließend werden die Hydrogel-Partikel mit einer wandgängigen Plexiglasscheibe überdeckt und ein wandgängiger Plexiglaszylinder mit Metallstiel eingeführt, wobei das Gesamtgewicht der Plexiglasscheibe und des Zylinders mit Stiel 100 g beträgt, so daß auf den Hydrogel-Partikeln ein Druck von 19,6 g/cm² lastet. Der Flüssigkeitsbehälter wird mit 0,9 Gew.-%iger Kochsalz-Lösung gefüllt. Dann wird die Meßzelle durch eine Bohrung des Deckels in die Flüssigkeit eingetaucht (Eintauchtiefe: 1,2 cm), wobei die Meßzelle durch den Tragring gehalten wird. Es können gleichzeitig bis zu 6 Meßzellen vermessen werden. Die Meßzellen werden 60 Minuten lang im Flüssigkeitsbehälter belassen, wobei die Hydrogel-Partikel unter Quellung Flüssigkeit entgegen der Schwerkraft unter zusätzlicher Gewichtsbelastung aufnehmen. Aufgrund der sehr hohen Flächenbelegung an Hydrogel-Partikeln sind zur Erzielung einer hohen Quellhöhe eine hohe Permeabilität der Gelschicht und eine hohe Kapillarität notwendig. Nach 60 Minuten wird die Meßzelle aus dem Flüssigkeitsbehälter genommen und die Höhe des gequollenen Gels bestimmt.

### Beispiele:

### Beispiel 1

In einem Ansatzkessel 1 wurde eine Mischung aus 367,7 kg entsalztem Wasser, 130,0 kg Acrylsäure, 1,0 kg Pentaerythritoltriallylether, 220 g 2,2'-Azobisamindinopropan-dihydrochlorid und 400 g Kaliumperoxodisulfat von Luftsauerstoff befreit und auf 4°C temperiert. In einem weiteren Ansatzkessel 2 wurde eine von Luftsauerstoff befreite Lösung von 40 g Ascorbinsäure in 20 kg Wasser hergestellt. Nach Fertigstellung der Lösungen wurde der Inhalt der beiden Ansatzkessel unter einem Druck von 1,5 bar im Stickstoffgegenstrom synchron in den Polymerisationsreaktor eingepreßt, wobei beide Lösungen mit Hilfe eines statischen Mischers vor Eintritt in den Reaktor gemischt wurden. Der Polymerisationsreaktor ist ein 600 l-Rohr-Reaktor mit einem Durchmesser von 0,50 m, der sich am Ende konusartig verjüngt. Der Rohrreaktor wurde dann verschlossen und die Reaktionslösung wird ohne Rühren stehengelassen, wobei durch einsetzende Polymerisation, in deren Verlauf die Temperatur bis auf ca. 86°C ansteigt, ein festes Gel entsteht. Man ließ über Nacht auf Raumtemperatur abkühlen und preßte danach am Kopf des Reaktors einen Stickstoffdruck von 6 bar auf. Nach Öffnen des Absperrventils, welches sich am Ende des Konus des Reaktors befindet, konnte das Gel mit Hilfe einer Pumpe ausgetragen werden, wobei das Gel durch die Wirkung der Pumpe zerkleinert wurde. Das Hydrogel wurde anschließend zusammen mit einer 50 gew.-%igen Lösung an Natriumhydroxid einem Wolf zugeführt, der wie folgt charakterisiert ist:

| | |
|---|---|
| Leistung | 5000 Wh/m³ |
| Frequenz des rotierenden Messers | 3 s⁻¹ |
| Energiedissipationsdichte | 750 W/l Mischvolumen |
| Verweilzeit des Hydrogels im Wolf | 25 s |
| freie Lochfläche der Lochscheibe | 30 % |
| Lochanfangsdurchmesser der Lochflächen | 8 mm (mit konischer Erweiterung unter einem Winkel von 15°), |

wobei die Mengenanteile Hydrogel und Natronlauge so gewählt wurden, daß ein durchschnittlicher Neutralisationsgrad der Acrylsäureeinheiten des Hydrogels von 74 Mol-% erreicht wurde. Die pH-Homogenität des einmal gewolften Hydrogels wurde durch Aufsprühen von pH-Indikator-Lösung überprüft. Die Hydrogel-Partikel wurden dann mit Hilfe eines Walzentrockners getrocknet, gemahlen und die Kornfraktion 106 - 850 µm ausgesiebt. In einem Lödige-Pflugscharmischer von 100 l Inhalt wurden 35 kg dieses Hydrogel-Pulvers vorgelegt. Im Verlauf von 5 bis 10 Minuten wurde eine Lösung von 35 g Ethylenglykoldiglycidylether, 1170 g Wasser und 580 g 1,2-Propandiol aufgedüst. Es wurde auf 120°C Produkttemperatur angeheizt und 60 Minuten bei dieser Temperatur gehalten, um das Lösungsmittel wieder abzudestillieren. Anschließend wurde abgekühlt, das Produkt ausgetragen und die Kornfraktion 120 - 850 µm abgesiebt. Man erhielt ein Produkt, das gekennzeichnet ist durch folgende physikalischen Daten, alle gemessen in 0,9 Gew.-%iger Kochsalz-Lösung:

| | | |
|---|---|---|
| CRC | = | 32 g/g |
| Extrahierbare Anteile (16 h) | = | 1,5 % |
| Vertikal-Absorption (1 g), Quellhöhe | = | 4,2 cm |

### Beispiel 2

Ein mit Aluminium beschichteter Tetrafluorethylen-Ethylen-Copolymerfilm wurde an der Oberfläche eines endlosen Bandes aus rostfreiem Stahl mit einer Breite von 450 mm und einer wirksamen Länge von 3000 mm so befestigt, daß die metallisierte Oberfläche mit der Bandoberfläche in Kontakt steht. Das endlose Band wurde in eine mit Stickstoff gefüllte Kammer, um die Sauerstoffkonzentration bei nicht mehr als 1 Vol.-% zu halten, eingeführt, während Sprüheinrichtungen so angeordnet waren, daß heißes oder kaltes Wasser auf die Rückseite des endlosen Bandes aufgesprüht werden konnte. Das endlose Band wurde mit einer Geschwindigkeit von 100 mm/min transportiert und Wasser von 15°C wird nach oben auf das Band aufgesprüht.

In einem Ansatzkessel 1 wurden 5080 Gewichtsanteile entsalztes Wasser vorgelegt, 669 Gewichtsanteile Natriumbicarbonat darin suspendiert und langsam ein Gemisch aus 2294 Gewichtsanteilen Acrylsäure und 8 Gewichtsanteilen Methylenbisacrylamid so zudosiert, daß ein Überschäumen der Reaktionslösung vermieden wurde, wobei sich diese auf eine Temperatur von ca. 3 - 5°C abkühlte. Bei einer Temperatur von 4°C wurden 2,2 Gewichtsanteile 2,2'-Azobisaminopropandihydrochlorid, gelöst in 20 Gewichtsanteilen entsalztem Wasser, und 4 Gewichtsanteile Kaliumperoxodisulfat, gelöst in 150 Gewichtsanteilen entsalztem Wasser, nacheinander zugegeben und gut verrührt. In einem zweiten Ansatzkessel 2 wurde ein Lösung von 0,4 Gewichtsanteilen Ascorbinsäure in 50 Gewichtsanteilen entsalztem Wasser hergestellt.

Die Lösungen aus Ansatzkessel 1 und 2 wurden dann im Verhältnis 80:1 über einen statischen Mischer kontinuierlich mit einer Rate von 135 1/h auf ein Ende des sich bewegenden Bandes aufgebracht.

Unter den obengenannten Bedingungen betrug die Zeit, innerhalb der die Monomerlösung der Polymerisation auf dem sich bewegenden Band unterworfen wurde, 30 Minuten und die Dicke der Monomerlösungsschicht auf dem Band betrug etwa 5 cm.

Am anderen Ende des endlosen Bandes wurde 30 Minuten nach Beginn der Zuführung der wäßrigen Monomerlösung ein Polymergel in Form eines Stranges mit einer Dicke von etwa 5 cm erhalten. Dieser Polymergelstrang wurde von der Bandoberfläche abgelöst und direkt in eine Schneideinrichtung vom Walzentyp eingeführt. Man erhielt so zerkleinerte Hydrogel-Teilchen, die zusammen mit 0,9 Gew.-% (bezogen auf Acrylsäure) Festsubstanz eines handelsüblichen kationischen Polyamidoaminharzes (KYMENE 557H® der Hercules Corp., USA), sowie mit einer 50 gew.-%igen Lösung an Natriumhydroxid dem in Beispiel 1 beschriebenem Wolf zugeführt wurden, wobei die Mengenanteile Hydrogel und Natronlauge so gewählt wurden, daß ein durchschnittlicher Neutralisationsgrad der Acrylsäureeinheiten des Hydrogels von 70 Mol-% erreicht wurde. Die pH-Homogenität des einmal gewolften Hydrogels wurde durch Aufsprühen von pH-Indikator-Lösung überprüft. Das gewolfte Hydrogel wurde dann heißluftgetrocknet, wobei die Lufttemperatur 175°C beträgt, die Luftgeschwindigkeit 1,5 m/s und die Verweilzeit im heißen Luftstrom 20 Minuten. Man erhielt ein Produkt, das nach Mahlung und Aussiebung der Kornfraktion 106 - 850 µm gekennzeichnet ist durch folgende physikalischen Daten, alle gemessen in 0,9 gew.-%iger Kochsalz-Lösung:

| | | |
|---|---|---|
| CRC | = | 29 g/g |
| Extrahierbare Anteile (16 h) | = | 1,2 % |
| Vertikal-Absorption (1 g), Quellhöhe | = | 3,8 cm |

### Beispiel 3

In einem Ansatzkessel 1 wurde eine Mischung aus 367,7 kg entsalztem Wasser, 130,0 kg Acrylsäure, 0,2 kg Allylmethacrylat, 0,5 kg Tetraallyloxyethan, 0,4 kg Divinyladipinat, 220 g 2,2'-Azobisaminopropandihydrochlorid und 350 g Kaliumperoxodisulfat von Luftsauerstoff befreit und auf 4°C temperiert. In einem weiteren Ansatzkessel 2 wurde eine von Luftsauerstoff befreite Lösung von 40 g Ascorbinsäure in 20 kg Wasser hergestellt. Nach Fertigstellung der Lösungen wurde der Inhalt der beiden Ansatzkessel unter einem Druck von 1,5 bar im Stickstoffgegenstrom synchron in den in Beispiel 1 beschriebenen Polymerisationsreaktor eingepreßt, wobei beide Lösungen mit Hilfe eines statischen Mischers vor Eintritt in den Reaktor gemischt wurden. Der Reaktor wurde dann verschlossen und die Reaktionslösung wurde ohne Rühren stehengelassen, wobei durch einsetzende Polymerisation, in deren Verlauf die Temperatur bis auf ca. 86°C ansteigt, ein festes Gel entstand. Man ließ über Nacht auf Raumtemperatur abkühlen und preßte danach am Kopf des Reaktors einen Stickstoffdruck von 6 bar auf. Nach Öffnen des Absperrventils, welches sich am Ende des Konus des Reaktors befindet, konnte das Gel mit Hilfe einer Pumpe ausgetragen werden, wobei das Gel durch die Wirkung der Pumpe zerkleinert wurde. Das Hydrogel wurde anschließend zusammen mit einer 50 gew.-%igen Lösung an Natriumhydroxid einem Wolf zugeführt, der wie folgt charakterisiert ist:

| | |
|---|---|
| Leistung | 4000 Wh/m³ |
| Frequenz des rotierenden Messers | 3 s⁻¹ |
| Energiedissipationsdichte | 6000 W/l Mischvolumen |
| Verweilzeit des Hydrogels im Wolf | 20 s |
| freie Lochfläche der Lochscheibe | 32 % |
| Lochanfangsdurchmesser der Lochflächen | 10 mm (mit konischer Erweiterung unter einem Winkel von 12°), |

wobei die Mengenanteile Hydrogel und Natronlauge so gewählt wurden, daß ein durchschnittlicher Neutralisationsgrad der Acrylsäureeinheiten des Hydrogels von 74 Mol-% erreicht wurde. Die pH-Homogenität des einmal gewolften Hydrogels wurde durch Aufsprühen von pH-Indikator-Lösung überprüft. Die Hydrogel-Partikel wurden dann mit Hilfe eines Walzentrockners getrocknet, gemahlen und die Kornfraktion 106 - 850 µm ausgesiebt. 6 kg des Hydrogel-Pulvers wurden in einem Petterson & Kelly-Mischer von 10 l Inhalt vorgelegt. Unter Mischen wurde im Verlauf von 5 Minuten eine Lösung aus 10 g Bisoxazolin, 12 g Aluminiumsulfat, 225 g i-Propanol und 225 g Wasser aufgedüst und 1 Minute nachgemischt. Das Produkt wurde anschließend im Trockenschrank 30 Minuten bei 185°C nachgetempert. Es war gekennzeichnet durch folgende physikalischen Daten, alle gemessen in 0,9 gew.-%iger Kochsalz-Lösung:

| | | |
|---|---|---|
| CRC | = | 35 g/g |
| Extrahierbare Anteile (16 h) | = | 3,2 % |
| Vertikal-Absorption (1 g), Quellhöhe | = | 4,0 cm |

### Beispiel 4

In einem Ansatzkessel 1 wurde eine Mischung aus 367,7 kg entsalztem Wasser, 130,0 kg Acrylsäure, 2,0 kg Polyethylenglykol-400-diallylether, 0,5 kg Triallyl-s-Triazin-2,4,6(1H,3H,5H)-trion, 220 g 2,2'-Azobisaminopropandihydrochlorid und 300 g Kaliumperoxodisulfat von Luftsauerstoff befreit und auf 2°C temperiert. In einem weiteren Ansatzkessel 2 wurde eine von Luftsauerstoff befreite Lösung von 40 g Ascorbinsäure in 20 kg Wasser hergestellt. Nach Fertigstellung der Lösungen wurde der Inhalt der beiden Ansatzkessel unter einem Druck von 1,5 bar im Stickstoffgegenstrom synchron in den in Beispiel 1 beschriebenen Polymerisationsreaktor eingepreßt, wobei beide Lösungen mit Hilfe eines statischen Mischers vor Eintritt in den Reaktor gemischt wurden. Der Reaktor wurde dann verschlossen und die Reaktionslösung wird ohne Rühren stehengelassen, wobei durch einsetzende Polymerisation, in deren Verlauf die Temperatur bis auf ca. 82°C ansteigt, ein festes Gel entsteht. Man ließ über Nacht auf Raumtemperatur abkühlen und preßte danach am Kopf des Reaktors einen Stickstoffdruck von 6 bar auf. Nach Öffnen des Absperrventils, welches sich am Ende des Konus des Reaktors befindet, konnte das Gel mit Hilfe einer Pumpe ausgetragen werden, wobei das Gel durch die Wirkung der Pumpe zerkleinert wurde. Das Hydrogel wurde anschließend zusammen mit 0,05 Gew.-% Oxazolidon bezogen auf Acrylsäure und einer 50 gew.-%igen Lösung an Natriumhydroxid dem in Beispiel 3 beschriebenen Wolf zugeführt, wobei die Mengenanteile Hydrogel und Natronlauge so gewählt wurden, daß ein durchschnittlicher Neutralisationsgrad der Acrylsäureeinheiten des Hydrogels von 72 Mol-% erreicht wurde. Die pH-Homogenität des einmal gewolften Hydrogels wurde durch Aufsprühen von pH-Indikator-Lösung überprüft. Die Hydrogel-Partikel wurden dann heißluftgetrocknet, wobei die Lufttemperatur 165°C, die Luftgeschwindigkeit 2 m/s und die Verweilzeit 20 Minuten betrug. Nach Mahlung und Aussiebung der Kornfraktion 106 - 850 µm wurden 35 kg dieses Hydrogel-Pulvers in einem Lödige-Pflugscharmischer von 100 l Inhalt vorgelegt. Im Verlauf von 5 bis 10 Minuten wurde eine Lösung von 70 g Polyglycerinpolyglycidylether (Denacol EX-5127 von Nagase Chemicals Ltd.), 10 g Zitronensäure, 1300 g Wasser und 3900 g Methanol aufgedüst. Es wurde auf 150°C Produkttemperatur angeheizt und 40 Minuten bei dieser Temperatur gehalten, um das Lösungsmittel wieder abzudestillieren. Anschließend wurde abgekühlt, das Produkt ausgetragen, mit 0,05 Gew.-% hydrophilem Silica (Aerosil 200) abgemischt und die Kornfraktion 120 - 850 µm abgesiebt. Man erhielt ein Produkt, das gekennzeichnet ist durch folgende physikalischen Daten, alle gemessen in 0,9 gew.-%iger Kochsalzlösung:

| | | |
|---|---|---|
| CRC | = | 30 g/g |
| Extrahierbare Anteile (16 h) | = | 2,0 % |
| Vertikal-Absorption (1 g), Quellhöhe | = | 5,3 cm. |

### Beispiel 5

In einem Ansatzkessel 1 wurden 5080 Gewichtsanteile entsalztem Wasser vorgelegt, 500 Gewichtsanteile Natriumbicarbonat darin suspendiert und langsam ein Gemisch aus 2412 Gewichtsanteilen Acrylsäure und 20 Gewichtsanteilen Allylmethacrylat so zudosiert, daß ein Überschäumen der Reaktionslösung vermieden wurde, wobei sich diese auf eine Temperatur von ca. 3 - 5°C abkühlt. Bei einer Temperatur von 4°C wurden 2,5 Gewichtsanteile 2,2'-Azobisaminopropandihydrochlorid, gelöst in 20 Gewichtsanteilen entsalztem Wasser, und 4 Gewichtsanteile Kaliumperoxodisulfat, gelöst in 150 Gewichtsanteilen entsalztem Wasser, nacheinander zugegeben und gut verrührt. In einem zweiten Ansatzkessel 2 wurde ein Lösung von 0,6 Gewichtsanteilen Ascorbinsäure in 50 Gewichtsanteilen entsalztem Wasser hergestellt.

Die Lösungen aus Ansatzkessel 1 und 2 wurden dann im Verhältnis 80:1 über einen statischen Mischer kontinuierlich mit einer Rate von 135 l/h auf ein Ende des sich bewegenden Bandes des in Beispiel 2 beschriebenen Reaktors aufgebracht.

Unter den obengenannten Bedingungen betrug die Zeit, innerhalb der die Monomerlösung der Polymerisation auf dem sich bewegenden Band unterworfen wurde, 30 Minuten und die Dicke der Monomerlösungsschicht auf dem Band betrug etwa 5 cm.

Am anderen Ende des endlosen Bandes wurde 30 Minuten nach Beginn der Zuführung der wäßrigen Monomerlösung ein Polymergel in Form eines Stranges mit einer Dicke von etwa 5 cm erhalten. Dieser Polymergelstrang wurde von der Bandoberfläche abgelöst und direkt in eine Schneideinrichtung vom Walzentyp eingeführt. Man erhielt so zerkleinerte Hydrogelteilchen, die zusammen mit pulverförmigem Natriumcarbonat dem in Beispiel 1 beschriebenen Wolf zugeführt wurden, wobei die Mengenanteile Hydrogel und Natriumcarbonat so gewählt wurden, daß ein durchschnittlicher Neutralisationsgrad der Acrylsäureeinheiten des Hydrogels von 60 Mol-% erreicht wurde. Anschließend wurden die Hydrogelpartikel in einem kontinuierlichen Drehrohr-Mischer mit 50 gew.-%iger Natronlauge besprüht, so daß sich ein End-Neutralisationsgrad der Acrylsäureeinheiten des Hydrogels von 70 Mol-% ergab. Man erhielt sehr lockere, flockige Gele mit separierten Gelteilchen, die heißluftgetrocknet wurden, wobei die Lufttemperatur 180°C betrug, die Luftgeschwindigkeit 2,5 m/s und die Verweilzeit 10 Minuten. Nach Mahlung und Aussiebung der Kornfraktion 106 - 850 µm wurden 35 kg des Hydrogel-Pulvers in einem Lödige-Pflugscharmischer von 100 1 Inhalt vorgelegt. Im Verlauf von 5 bis 10 Minuten wurde gleichzeitig über zwei Düsen eine Lösung von 50 g KYMENE 557H®, 600 g Wasser und 600 g 1,2-Propandiol sowie eine Lösung von 14 g Ethylenglykoldiglycidylether, 53 g Aluminiumsulfat, 840 g Wasser und 360 g 1,2-Propandiol aufgedüst. Es wurde auf 170°C Produkttemperatur angeheizt und 15 Minuten bei dieser Temperatur gehalten, um das Lösungsmittel wieder abzudestillieren. Anschließend wurde abgekühlt, das Produkt ausgetragen und die Kornfraktion 120 - 850 µm abgesiebt. Man erhielt ein Produkt, das gekennzeichnet ist durch folgende physikalischen Daten, alle gemessen in 0,9 gew.-%iger Kochsalz-Lösung:

| | | |
|---|---|---|
| CRC | = | 24 g/g |
| Extrahierbare Anteile (16 h) | = | 1,8 % |
| Vertikal-Absorption (3 g), Quellhöhe | = | 5,4 cm |

Die nach den Beispielen 1 bis 5 erhaltenen Hydrogele zeichnen sich durch hervorragendes Absorptionsvermögen bei hoher Kapillarität und niedrigen Gehalten an Extrahierbaren aus, und sind deshalb in hervorragender Weise als Absorptionsmittel für Wasser und wäßrige Flüssigkeiten, insbesondere von Körperflüssigkeiten, wie z.B. Urin oder Blut geeignet, beispielsweise in Hygieneartikeln wie z.B. Baby- und Erwachsenenwindeln, Damenbinden, Tampons und dergleichen.

## Patentansprüche

1. Hydrophiles, hochquellfähiges Hydrogel auf Basis (co)polymerisierter Monomerer oder auf Basis von Pfropf(co)polymerer, hergestellt durch
a) radikalische (Co)polymerisation von einem oder mehreren hydrophilen Monomeren der Formel 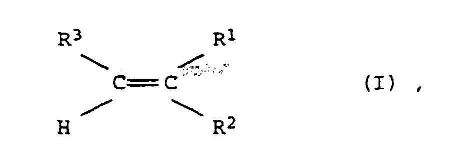 worin
R¹ Wasserstoff, Methyl oder Ethyl,
R² eine Gruppe -COOR⁴, Hydroxysulfonyl, Phosphonyl, die mit (C₁ - C₄)-Alkanol veresterte Phosphonylgruppe oder eine Gruppe der allgemeinen Formel (II) 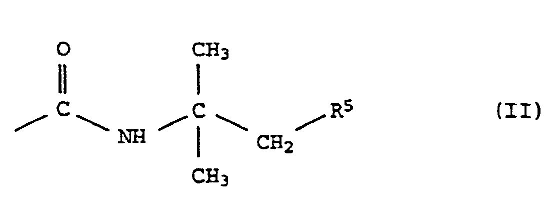
R³ Wasserstoff, Methyl, Ethyl oder die Carboxylgruppe,
R⁴ Wasserstoff, Amino-(C₁-C₄)-alkyl oder Hydroxy-(C₁-C₄)-alkyl und
R⁵ Hydroxysulfonyl, Phosphonyl oder Carboxyl bedeuten,
oder Pfropf(co)polymerisation von einem oder mehreren hydrophilen Monomeren der Formel auf eine Pfropfgrundlage, wobei der durchschnittliche Neutralisationsgrad der säuregruppenhaltigen Monomeren 0 bis 40 Mol-% beträgt
b) Zerkleinerung des sauren Hydrogels
c) Neutralisation des sauren Hydrogels auf einen End-Neutralisationsgrad von 50 - 85 Mol-% durch Zugabe eines Neutralisationsmittels
d) Trocknung, Mahlung und Siebung der Hydrogelteilchen
**dadurch gekennzeichnet, daß** das Hydrogel
- eine Zentrifugenretention für eine 0,9 %ige wäßrige NaCl-Lösung von mindestens 29 g/g und
- eine Quellhöhe bei der Vertikalabsorption (1 g) von mindestens 3,5 cm
- einen Gehalt an Extrahierbaren (16 h-Wert) kleiner als 5 % aufweist
oder
- eine Zentrifugenretention für eine 0,9 %ige wäßrige NaCl-Lösung von mindestens 23 g/g und
- eine Quellhöhe bei der Vertikalabsorption (3 g) von mindestens 5 cm
- einen Gehalt an Extrahierbaren (16 h-Wert) kleiner 4 % aufweist.

2. Hydrophiles, hochquellfähiges Hydrogel nach Anspruch 1, **dadurch gekennzeichnet, daß** die Polymerisation in Ruhe erfolgt.

3. Hydrophiles, hochquellfähiges Hydrogel nach Anspruch 1 und/oder 2, **dadurch gekennzeichnet, daß** hydrophile Monomere Verbindungen der Formel (I) 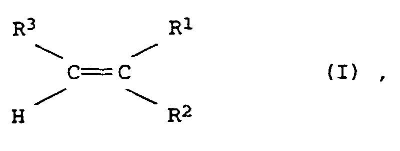 worin
R¹ Wasserstoff, Methyl oder Ethyl,
R² eine Gruppe -COOR⁴, Hydroxysulfonyl, Phosphonyl, die mit (C₁-C₄)-Alkanol veresterte Phosphonylgruppe oder eine Gruppe der allgemeinen Formel (II) 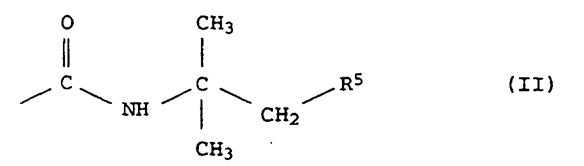
R³ Wasserstoff, Methyl, Ethyl oder die Carboxylgruppe,
R⁴ Wasserstoff, Amino-(C₁-C₄)-alkyl oder Hydroxy-(C₁-C₄)-alkyl und
R⁵ Hydroxysulfonyl, Phosphonyl oder Carboxyl bedeuten, sind.

4. Hydrophiles, hochquellfähiges Hydrogel nach einem oder mehreren der Ansprüche 1 bis 3, **dadurch gekennzeichnet, daß** geeignete Pfropfgrundlagen Stärke, Stärkederivate, Cellulose, Cellulosederivate, Polyvinylalkohol, Polyalkylenoxid, Polyethylenoxid, Polypropylenoxid und hydrophile Polyester sind.

5. Hydrophiles, hochquellfähiges Hydrogel nach einem oder mehreren der Ansprüche 1 bis 4, **dadurch gekennzeichnet, daß** die (Co)polymerisation der hydrophilen Monomeren in Anwesenheit von Vernetzern durchgeführt wird.

6. Hydrophiles, hochquellfähiges Hydrogel nach einem oder mehreren der Ansprüche 1 bis 5, **dadurch gekennzeichnet, daß** die Neutralisation des sauren Hydrogels auf einen End-Neutralisationsgrad von 50 - 85 Mol-% durch Vermischung mit einem Neutralisationsmittel in einem Wolf mittels einem System aus Schnecke, rotierendem Messer, Stauzone und Lochscheibe erfolgt, wobei
a) die Leistung des Wolfes 1000 bis 6000 Wh/m³ beträgt
b) das Hydrogel durch eine Zone mit einer Energiedissipationsdichte von 400 bis 800 W/l Mischvolumen geführt wird
c) die mittlere Verweilzeit des Hydrogels im Wolf 5 bis 30 Sekunden beträgt
d) die freie Lochfläche der Lochscheibe 20 bis 40 % beträgt
und daß die nachneutralisierten Hydrogelteilchen ohne weitere Scherbelastung dem Trocknungsschritt unterworfen werden.

7. Hydrophiles, hochquellfähiges Hydrogel nach einem oder mehreren der Ansprüche 1 bis 6, **dadurch gekennzeichnet, daß** bei der Neutralisation des Gels zusammen mit dem Neutralisationsmittel noch ein oder mehrere andere Stoffe, die mit freier Acrylsäure und/oder mit den Carboxylgruppen des Hydrogels reagieren können, und/oder Feinkorn von wasserquellbaren hydrophilen Polymeren zugesetzt wird.

8. Hydrophiles, hochquellfähiges Hydrogel nach einem oder mehreren der Ansprüche 1 bis 7, **dadurch gekennzeichnet, daß** das Hydrogel eine Zentrifugenretention für eine 0,9 %ige wäßrige NaCl-Lösung von mindestens 31 g/g aufweist.

9. Hydrophiles, hochquellfähiges Hydrogel nach einem oder mehreren der Ansprüche 1 bis 8, **dadurch gekennzeichnet, daß** das Hydrogel eine Quellhöhe bei der Vertikalabsorption (1 g) von mindestens 4,0 cm aufweist.

10. Hydrophiles, hochquellfähiges Hydrogel nach einem oder mehreren der Ansprüche 1 bis 9, **dadurch gekennzeichnet, daß** die Nachneutralisation in zwei Schritten durchgeführt wird, wobei der erste Neutralisationsschritt im Wolf stattfindet und der zweite Neutralisationsschritt durch Aufbringen des Neutralisationsmittels ohne mechanische Scherbelastung auf die Hydrogelpartikel erfolgt.

11. Hydrophiles, hochquellfähiges Hydrogel nach einem oder mehreren der Ansprüche 1 bis 13, **dadurch gekennzeichnet, daß** die getrockneten und gemahlenen Hydrogelteilchen kovalent oberflächennachvernetzt werden.

12. Hydrophiles, hochquellfähiges Hydrogel nach einem oder mehreren der Ansprüche 1 bis 14, **dadurch gekennzeichnet, daß** die Oberfläche der getrockneten und gemahlenen Hydrogelpartikel durch die Bildung von Metallkomplexen modifiziert wird.

13. Verwendung der Hydrogele gemäß einem oder mehreren der Ansprüche 1 bis 12 als Absorptionsmittel für Wasser und wäßrige Flüssigkeiten.

14. Verwendung nach Anspruch 13 zur Absorption von Körperflüssigkeiten, insbesondere in Hygieneartikeln, wie z.B. Windeln, Tampons oder Damenbinden.

## Claims

1. A hydrophilic, highly swellable hydrogel based on (co)polymerized monomers or based on graft (co)polymers and prepared by
a) free-radically (co)polymerizing one or more hydrophilic monomers 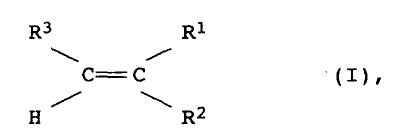 where
R¹ is hydrogen, methyl or ethyl,
R² is -COOR⁴, hydroxysulfonyl, phosphonyl, a (C₁-C₄)-alkanol-esterified phosphonyl group or a group of the general formula (II) 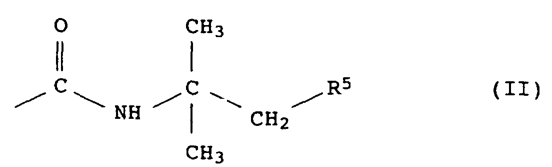
R³ is hydrogen, methyl, ethyl or carboxyl,
R⁴ is hydrogen, amino-(C₁-C₄)-alkyl or hydroxy-(C₁-C₄)-alkyl, and
R⁵ is hydroxysulfonyl, phosphonyl or carboxyl,
or graft (co)polymerizing one or more hydrophilic monomers of the formula I onto a grafting base, the average degree of neutralization of the acid-functional monomers being from 0 to 40 mol%,
b) comminuting the acidic hydrogel,
c) neutralizing the acidic hydrogel to an ultimate degree of neutralization of 50-85 mol% by adding a neutralizing agent,
d) drying, grinding and sieving the hydrogel particles
**characterized by**
- a centrifuge retention of at least 29 g/g for a 0.9% aqueous NaCl solution and
- a vertical absorption (1 g) swell height of at least 3.5 cm
- an extractables content (16 h value) of less than 5%
or
- a centrifuge retention of at least 23 g/g for a 0.9% aqueous NaCl solution and
- a vertical absorption (3 g) swell height of at least 5 cm
- an extractables content (16 h value) of less than 4%.

2. The hydrogel of claim 1, **characterized in that** the polymerization is carried out without agitation.

3. The hydrogel of claim 1 and/or 2, **characterized in that** hydrophilic monomers are compounds of the formula (I) 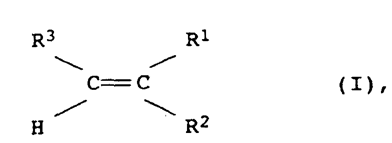 where
R¹ is hydrogen, methyl or ethyl,
R² is -COOR⁴, hydroxysulfonyl, phosphonyl, a (C₁-C₄)-alkanol-esterified phosphohyl group or a group of the general formula (II) 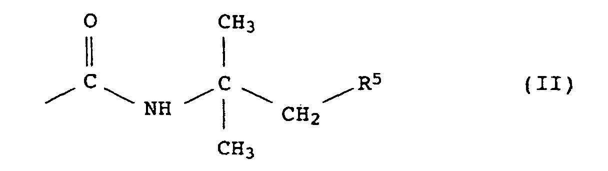
R³ is hydrogen, methyl, ethyl or carboxyl,
R⁴ is hydrogen, amino-(C₁-C₄)-alkyl or hydroxy-(C₁-C₄)-alkyl, and
R⁵ is hydroxysulfonyl, phosphonyl or carboxyl.

4. The hydrogel of one or more of claims 1 to 3, **characterized in that** suitable grafting bases are selected from the group consisting of starch, starch derivatives, cellulose, cellulose derivatives, polyvinyl alcohol, polyalkylene oxide, polyethylene oxide, polypropylene oxide and hydrophilic polyesters.

5. The hydrogel of one or more of claims 1 to 4, **characterized in that** the (co)polymerizing of the hydrophilic monomers is effected in the presence of crosslinkers.

6. The hydrogel of one or more of claims 1 to 5, **characterized in that** the acidic hydrogel is neutralized to an ultimate degree of neutralization of 50-85 mol% by mixing with a neutralizing agent in a mincer comprising a system of screw, rotating blade, restricted flow zone and breaker plate, wherein
a) the mincer has a power output of from 1 000 to 6 000 Wh/m³,
b) the hydrogel is passed through a zone having an energy dissipation intensity of from 400 to 800 W/l of mixing volume,
c) the average hydrogel residence time in the mincer is from 5 to 30 seconds,
d) the open area of the breaker plate is from 20 to 40%
and **in that** the postneutralized hydrogel particles are subjected to the drying step without further shearing.

7. The hydrogel of one or more of claims 1 to 6, **characterized in that**, as well as the neutralizing agent, one or more other reactive materials capable of reacting with free acrylic acid and/or with the carboxyl groups of the hydrogel and/or water-swellable hydrophilic polymer fines are added during the neutralization of the gel.

8. The hydrogel of one or more of claims 1 to 7, **characterized by** a centrifuge retention of at least 31 g/g for a 0.9% aqueous NaCl solution.

9. The hydrogel of one or more of claims 1 to 8, **characterized by** a vertical absorption (1 g) swell height of at least 4.0 cm.

10. The hydrogel of one or more of claims 1 to 9, **characterized in that** the postneutralization is carried out in two steps, the first neutralization step taking place in the mincer and the second neutralization step being effected by applying the neutralizing agent to the hydrogel particles without mechanical shearing stress.

11. The hydrogel of one or more of,claims 1 to 13, **characterized in that** the dried and ground hydrogel particles are covalently surface postcrosslinked.

12. The hydrogel of one or more of claims 1 to 14, **characterized in that** the surface of the dried and ground hydrogel particles is modified by the formation of metal complexes.

13. The use of the hydrogels of one or more of claims 1 to 12 as absorbents for water and aqueous fluids.

14. The use according to claim 13 for absorbing body fluids, especially in hygiene articles such as diapers, tampons or sanitary napkins.

## Revendications

1. Hydrogel hydrophile à forte capacité de gonflement, à base de monomères (co)polymérisés ou à base de (co)polymères greffés, préparé par
a) (co)polymérisation radicalaire d'un ou plusieurs monomères hydrophiles de formule 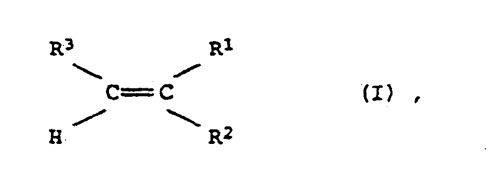 dans laquelle
R¹ représente de l'hydrogène ou un radical méthyle ou éthyle,
R² représente un groupe -COOR⁴, hydroxysulfonyle, phosphonyle, les groupes phosphonyle estérifiés avec un alcanol en C₁ à C₄ ou un groupe de formule générale (II) 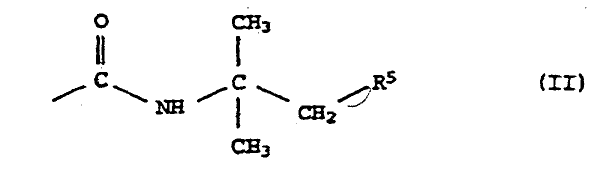
R³ représente de l'hydrogène ou un radical méthyle, éthyle ou le groupe carboxyle,
R⁴ représente de l'hydrogène ou un radical amino(C₁-C₄)alkyle ou hydroxy(C₁-C₄)alkyle, et
R⁵ représente un groupe hydroxysulfonyle, phosphonyle ou carboxyle,
ou (co)polymérisation de greffage d'un ou plusieurs monomères hydrophiles de formule (I) sur une base de greffage, le degré moyen de neutralisation des monomères contenant des groupes d'acide étant de 0 à 40% molaires,
b) fragmentation de l'hydrogel acide,
c) neutralisation de l'hydrogel acide à un degré de neutralisation final de 50 à 85% molaires par addition d'un agent de neutralisation,
d) séchage, mouture et criblage des particules d'hydrogel,
**caractérisé en ce que** l'hydrogel présente
- une rétention centrifuge pour une solution aqueuse de NaCl à 0,9% d'au moins 29 g/g et
- une hauteur de gonflement en absorption verticale (1 g) d'au moins 3,5cm, une teneur en fractions extractibles (valeur 16 h) de moins de 5%,
ou
- une rétention centrifuge pour une solution aqueuse de NaCl à 0,9% d'au moins 23 g/g et
- une hauteur de gonflement en absorption verticale (1 g) d'au moins 5cm,
- une teneur en fractions extractibles (valeur 16 h) de moins de 4%.

2. Hydrogel hydrophile à forte capacité de gonflement selon la revendication 1, **caractérisé en ce que** la polymérisation est réalisée au repos.

3. Hydrogel hydrophile à forte capacité de gonflement selon la revendication 1 et/ou 2, **caractérisé en ce que** les monomères hydrophiles sont des composés de la formule (I) 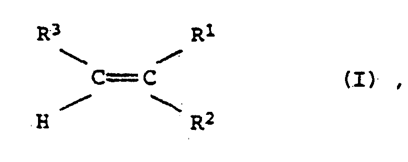 dans laquelle
R¹ représente de l'hydrogène ou un radical méthyle ou éthyle,
R² représente un groupe -COOR⁴, hydroxysulfonyle, phosphonyle, les groupes phosphonyle estérifiés avec un alcanol en C₁ à C₄ ou un groupe de formule générale (II) 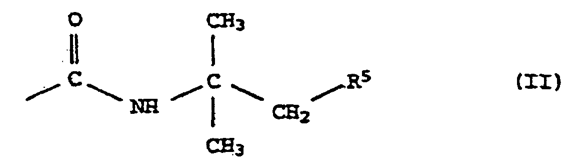
R³ représente de l'hydrogène ou un radical méthyle, éthyle ou le groupe carboxyle,
R⁴ représente de l'hydrogène ou un radical amino(C₁-C₄)alkyle ou hydroxy(C₁-C₄)alkyle, et
R⁵ représente un groupe hydroxysulfonyle, phosphonyle ou carboxyle.

4. Hydrogel hydrophile à forte capacité de gonflement selon une ou plusieurs des revendications 1 à 3, **caractérisé en ce que** des bases de greffage appropriées sont des amidons, des dérivés d'amidon, de la cellulose, des dérivés de cellulose, du poly(alcool vinylique), du poly(oxyde d'alkylène), du poly(oxyde d'éthylène), du poly(oxyde de propylène) et des polyesters hydrophiles.

5. Hydrogel hydrophile à forte capacité de gonflement selon une ou plusieurs des revendications 1 à 4, **caractérisé en ce que** la (co)polymérisation des monomères hydrophiles est entreprise en présence d'agents réticulants.

6. Hydrogel hydrophile à forte capacité de gonflement selon une ou plusieurs des revendications 1 à 5, **caractérisé en ce que** la neutralisation de l'hydrogel acide jusqu'à un degré final de neutralisation de 50 à 85% molaires est réalisée par mélange avec un agent de neutralisation, dans un hachoir, au moyen d'un système constitué d'une vis, d'un couteau rotatif, d'une zone de retenue et d'un disque perforé, où
a) la puissance du hachoir est de 1000 à 6000 Wh/m³,
b) l'hydrogel est guidé à travers une zone avec une densité de dissipation d'énergie de 400 à 800 W/I de volume de mélange,
c) la durée de séjour moyenne de l'hydrogel dans le hachoir est de 5 à 30 secondes,
d) la surface perforée libre du disque perforé est de 20 à 40%,
et **en ce que** les particules d'hydrogel post-neutralisées sont soumises à l'étape de séchage sans autre sollicitation de cisaillement.

7. Hydrogel hydrophile à forte capacité de gonflement selon une ou plusieurs des revendications 1 à 6, **caractérisé en ce que**, lors de la neutralisation du gel, on ajoute encore, en même temps que l'agent de neutralisation, une ou plusieurs autres substances capables de réagir avec l'acide acrylique libre et/ou avec les groupes carboxyle de l'hydrogel, et/ou une fine mouture de polymères hydrophiles gonflant à l'eau.

8. Hydrogel hydrophile à forte capacité de gonflement selon une ou plusieurs des revendications 1 à 7, **caractérisé en ce que** l'hydrogel présente une rétention centrifuge pour une solution aqueuse de NaCl à 0,9% d'au moins 31 g/g.

9. Hydrogel hydrophile à forte capacité de gonflement selon une ou plusieurs des revendications 1 à 8, **caractérisé en ce que** l'hydrogel présente une hauteur de gonflement en absorption verticale (1 g) d'au moins 4,0 cm.

10. Hydrogel hydrophile à forte capacité de gonflement selon une ou plusieurs des revendications 1 à 9, **caractérisé en ce que** la post-neutralisation est réalisée en deux étapes, la première étape de neutralisation ayant lieu dans le hachoir et la deuxième étape de neutralisation étant réalisée par application de l'agent de neutralisation sur les particules d'hydrogel, sans contrainte mécanique de cisaillement.

11. Hydrogel hydrophile à forte capacité de gonflement selon une ou plusieurs des revendications 1 à 10, **caractérisé en ce que** les particules d'hydrogel séchées et moulues sont réticulées en surface de manière covalente.

12. Hydrogel hydrophile à forte capacité de gonflement selon une ou plusieurs des revendications 1 à 11, **caractérisé en ce que** la surface des particules d'hydrogel séchées et moulues est modifiée par la formation de complexes métalliques.

13. Utilisation des hydrogels selon une ou plusieurs des revendications 1 à 12 comme milieux d'absorption pour de l'eau et des liquides aqueux.

14. Utilisation selon la revendication 13 pour l'absorption de liquides corporels, en particulier dans des articles d'hygiène comme par exemple des couches, des tampons ou des serviettes hygiéniques.
